# FASCICULE DE BREVET EUROPEEN

(11) **EP 0 643 992 B1**
(45) Date de publication et mention de la délivrance du brevet: **12.03.1997**
(21) Numéro de dépôt: 94401978.5
(22) Date de dépôt: 06.09.1994
(51) Int. Cl.: B01J 27/053, B01J 27/02, B01J 21/08, B01J 35/10, C07C 2/62

(54) **Catalyseur d'alkylation d'isoparaffine C4-C5 par au moins une oléfine C3-C6**
Katalysator für die Alkylierung eines C4-C5 Isoparaffins mit mindestens einem C3-C6 Olefin
Catalyst for the alkylation of a C4-C5 isoparaffin with at least one C3-C6 olefin

(30) Priorité: 10.09.1993 FR 9310896; 14.12.1993 FR 9314994
(43) Date de publication de la demande: 22.03.1995
(73) Titulaire: INSTITUT FRANCAIS DU PETROLE, 92502 Rueil-Malmaison (FR)
(72) Inventeur: Joly, Jean-François, F-75003 Paris (FR); Marcilly, Christian, F-78800 Houilles (FR); Benazzi, Eric, F-78360 Montesson (FR); Chaigne, Frédéric, F-85230 Bauvoir S/Mer (FR); Bernhard, Jean-Yves, F-91540 Mennecy (FR)

(56) Documents cités:
- EP-A- 0 067 467
- EP-A- 0 325 811
- EP-A- 0 539 277

## Description

La présente invention concerne un catalyseur comprenant de la silice et une phase acide comportant de l'acide sulfurique et son utilisation en alkylation catalytique d'isobutane et/ou d'isopentane au moyen d'au moins une oléfine, qui permet d'obtenir au moins un produit par exemple dans le groupe constitué par les diméthylbutanes, les triméthylpentanes, les triméthylhexanes et les triméthylheptanes.

On sait que, pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation d'au moins une isoparaffine (isobutane et/ou isopentane) par au moins une oléfine contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent soit l'acide sulfurique soit l'acide fluorhydrique comme catalyseur. Dans ces procédés le catalyseur acide constitue une phase liquide qui est mise en contact avec le mélange isobutane-oléfine liquide pour former une émulsion. Ces procédés sont coûteux et posent d'importants problèmes vis-à-vis de la sécurité des personnes et de l'environnement. Afin de remédier à ces problèmes, des systèmes catalytiques différents des acides sulfurique et fluorhydrique en phase liquide ont été recherchés.

La demande de brevet européen EP-A-0539277 décrit un catalyseur contenant de la silice et une phase acide solide comprenant de l'acide sulfurique, la silice selon ladite demande de brevet est telle que son volume poreux est compris entre 0,005 et 1,5 cm³/g, et sa surface spécifique est comprise entre 0,01 et 1 500 m²/g. Ladite phase acide comprend éventuellement un additif choisi dans le groupe formé par H₃PO₄, B(OH)₃, BF₄H, FSO₃H, CF₃CO₂H, SbF₅, CF₃SO₃H et SO₃.

Les demandes de brevet européen EP-A-0542612 et EP-A-0542620 décrivent le même type de catalyseur, mais avec des supports différents : le support de catalyseur décrit dans EP-A-0542612 comprend au moins un oxyde sulfaté et le support de catalyseur décrit dans EP-A-0542620 est choisi parmi les résines, le charbon, les zéolithes, les oxydes tels que ZrO₂, T₂O₂, Al₂O₃, Fe₂O₃, HfO₂ et les catalyseurs de craquage usagés.

La présente invention concerne un catalyseur pour l'alkylation d'au moins une isoparaffine (isobutane et/ou isopentane) avec au moins une oléfine comprenant de 3 à 6 atomes de carbone par molécule. Plus précisément, l'invention concerne un catalyseur comprenant de la silice et une phase acide comportant de l'acide sulfurique, la silice ayant été imprégnée par ladite phase acide, sa préparation et son utilisation en alkylation catalytique d'isobutane et/ou d'isopentane en présence d'au moins une oléfine comportant de 3 à 6 atomes de carbone par molécule, ledit catalyseur étant caractérisé en ce qu'il est constitué essentiellement de particules de diamètre moyen compris entre 0,1 et 150 µm (1µm = 10⁻⁶m) de préférence entre 5 et 110 µm et de manière encore plus préférée entre 5 et 80 µm, et en ce que la silice, avant son imprégnation par ladite phase acide, a un volume poreux total supérieur à 1,5 cm³/g, de préférence compris entre 1,5 et 6 cm³/g.

Le catalyseur selon la présente invention conduit à des performances catalytiques améliorées par rapport à celles décrites dans la demande de brevet européen EP-A-0539277.

Le catalyseur selon l'invention est tel que sa teneur pondérale en phase acide est généralement supérieure à 70 %.

La silice peut éventuellement contenir des impuretés comme par exemple les oxydes, les alcalins, les alcalino-terreux, les composés d'aluminium ou toute autre impureté connue de l'homme du métier, la quantité totale de ces impuretés n'excédant généralement pas 2 % en poids par rapport à la silice.

Le titre de l'acide sulfurique est avantageusement compris entre 90 et 100 % en poids, de préférence entre 97 et 100 % en poids et de manière encore plus préférée entre 98 et 100 % en poids.

Il est possible d'ajouter dans la phase acide comportant de l'acide sulfurique, avant l'imprégnation, au moins un additif visant à améliorer les performances catalytiques.

L'additif est généralement choisi dans le groupe formé par H₃PO₄, B(OH)₃, HB(HSO₄)₄, BF₄H, FSO₃H, CF₃CO₂H, SbF₅, CF₃SO₃H et SO₃ ; de préférence, l'additif est l'acide trifluorométhane sulfonique CF₃SO₃H ou l'anhydride sulfurique SO₃, mais tout additif connu de l'homme du métier est envisageable.

La teneur totale de la phase acide en additif(s) dépend de nombreux paramètres parmi lesquels on peut citer la nature de l'additif. Par exemple, lorsque l'additif est l'acide trifluorométhane sulfonique CF₃SO₃H, la teneur totale en additif dans la phase acide est généralement comprise entre 0,1 et 50% poids, de préférence entre 0,1 et 30% poids, et de manière encore plus préféré entre 5 et 15 % poids.

La phase acide occupe entre 80 et 100 % du volume poreux total de la silice, et de préférence entre 90 et 100 % dudit volume poreux.

La silice est généralement telle que, avant son imprégnation par la phase acide, sa surface spécifique est comprise entre 0,1 et 1 500 m²/g. De plus, elle est généralement constituée essentiellement de particules de diamètre moyen compris entre 0,1 et 150 µm.

Le procédé de préparation du catalyseur selon l'invention comprend deux étapes. Dans une première étape la silice est calcinée à une température supérieure à 50 °C, de préférence supérieure à 80 °C et de manière encore plus préférée comprise entre 150 et 600°C, par exemple égale à environ 500° C. La durée de cette étape de calcination est habituellement comprise entre 10 minutes et 50 heures, de préférence entre 15 minutes et 25 heures. La calcination peut être effectuée en présence d'air sec ou de mélange air sec/azote, de débit compris entre 0,001 et 10 l/h/g, de préférence entre 0,1 et 5 l/h/g. La seconde étape consiste en l'imprégnation de ladite silice calcinée par la phase acide. Pour réaliser cette étape on peut utiliser toutes les techniques bien connues de l'homme du métier. A ce procédé de préparation peut s'ajouter une étape de préparation de la phase acide, préalable à l'étape d'imprégnation.

Le catalyseur selon l'invention ainsi préparé n'a subi aucune calcination postérieure à l'étape d'imprégnation. Lorsqu'il est utilisé en alkylation d'isoparaffine par au moins une oléfine, il ne subit avant son utilisation aucune calcination et ainsi entre l'étape d'imprégnation et ladite utilisation, il n'a subi aucune calcination. Le catalyseur selon l'invention ainsi préparé est donc immédiatement prêt à l'emploi.

Le catalyseur selon la présente invention est mis en oeuvre dans un procédé qui permet de réaliser dans les meilleures conditions la réaction d'alkylation d'au moins une isoparaffine par au moins une oléfine. En particulier, ladite réaction étant caractérisée par une forte exothermicité (environ 83,6 kJ/mol de butène transformé), le procédé dans lequel est mis en oeuvre le catalyseur selon la présente invention permet d'obtenir une bonne homogénéité de température et de concentration en réactifs.

Dans le procédé d'alkylation de l'isoparaffine utilisant le catalyseur selon la présente invention, les conditions opératoires, et plus particulièrement la température et la pression, sont généralement choisies de façon à ce que le mélange constitué par l'(les) isoparaffine(s), l'(les) oléfine(s) et les produits de la réaction soit liquide. De plus, il est important que le catalyseur soit immergé dans ledit liquide de façon à assurer partout un bon contact liquide-solide.

Le catalyseur selon la présente invention est avantageusement mis en oeuvre dans la zone réactionnelle d'alkylation de l'isoparaffine avec au moins une oléfine comprenant de 3 à 6 atomes de carbone par molécule, en phase liquide et en mélange avec l'isoparaffine ou un mélange d'isoparaffines. Le catalyseur selon l'invention peut être mis en oeuvre en lit expansé, en zone réactionnelle presque parfaitement agitée ou en lit circulant, et de préférence il est mis en oeuvre dans un procédé qui utilise une phase liquide continue, le catalyseur étant utilisé sous forme de suspension par exemple selon les deux mises en oeuvre décrites ci-après.

Dans le cas où le catalyseur est utilisé sous forme d'une suspension, on peut utiliser, dans une première mise en oeuvre une zone réactionnelle à mélange presque parfait, c'est-à-dire à mélange parfait ou s'en rapprochant (cuve agité ou Grignard), utilisant au moins un moyen d'agitation par exemple au moins une hélice, de façon à obtenir une agitation suffisante du catalyseur en suspension dans la phase liquide hydrocarbonée, laquelle comprend généralement l'(les) isoparaffine(s) (isobutane et/ou isopentane), au moins une oléfine, éventuellement au moins un diluant inerte (par exemple le propane et le normal butane) et les produits de la réaction d'alkylation. La charge à convertir, constituée d'isobutane et/ou d'isopentane et d'au moins une oléfine, peut être par exemple introduite sous forme liquide en au moins un point au sein de la phase liquide hydrocarbonée présente dans la zone réactionnelle.

Une deuxième mise en oeuvre du catalyseur selon la présente invention en suspension dans une phase hydrocarbonée est le lit mobile à co-courant ou le lit circulant. Dans cette mise en oeuvre le catalyseur en suspension dans la phase liquide hydrocarbonée, comprenant généralement l'isobutane et/ou l'isopentane, au moins une oléfine, éventuellement au moins un diluant inerte (par exemple le propane ou le normal butane) et les produits de la réaction d'alkylation, circule de bas en haut dans la zone réactionnelle. L'ensemble constitué par la suspension de catalyseur dans la phase hydrocarbonée circule ensuite au travers d'au moins un échangeur de chaleur et d'au moins une pompe, avant d'être de nouveau introduit à l'entrée de la zone réactionnelle. La charge à convertir, constituée d'isobutane et/ou d'isopentane et d'au moins une oléfine, est introduite soit sous forme liquide, soit sous forme gazeuse en au moins un point de la zone réactionnelle.

Dans les deux types de mises en oeuvre décrites précédemment, l'isoparaffine (isobutane et/ou isopentane) n'ayant pas été convertie ou ayant été introduite en excès par rapport à la stoechiométrie de la réaction est généralement recyclée après séparation de l'alkylat, soit par introduction directe dans la zone réactionnelle, soit par mélange avec la charge à convertir.

Le mélange isoparaffine(s)-oléfine(s) est généralement introduit dans la zone réactionnelle à une vitesse spatiale horaire, exprimée en poids d'oléfine introduite par unité de poids du catalyseur et par heure (pph) comprise entre 0,001 et 10 h⁻¹ et de préférence comprise entre 0,002 et 2 h⁻¹. Ledit mélange peut aussi être réalisé à l'intérieur de la zone réactionnelle. Dans tous les cas le mélange ainsi constitué est dans la zone réactionnelle dans des conditions de pression et de température telles que le mélange d'hydrocarbures reste liquide sur le catalyseur.

La température de réaction est inférieure à +10 °C, de préférence à 0 °C et de manière souvent plus préférée inférieure à -3 °C. La pression de la zone réactionnelle est suffisante pour maintenir les hydrocarbures à l'état liquide dans ladite zone.

Afin de limiter les réactions secondaires, on peut utiliser un excès d'isoparaffine(s) par rapport à l'(les)oléfine(s). A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butène(s) dans la charge est généralement compris entre 1 et 100, de préférence entre 3 et 50 et de manière souvent préférée entre 5 et 15.

Lorsque la nature du catalyseur et les conditions de réaction sont judicieusement choisies (en particulier la température), le catalyseur selon l'invention permet la production de produits d'alkylation d'au moins une isoparaffine par au moins une oléfine qui sont intéressants comme carburants pour les moteurs et constituants d'essence et qui comprennent par exemple au moins 60 % moles de paraffines possédant 8 atomes de carbone par molécule et moins de 1 % moles de composés non saturés, les paraffines comprenant 8 atomes de carbone par molécule comportant 70 à 98 % en moles de triméthylpentanes.

Un autre avantage du catalyseur selon la présente invention est la possibilité d'alkyler, à basse température, l'isobutane avec des mélanges d'oléfines comportant de 3 à 6 atomes de carbone par molécule, où la proportion d'oléfines comportant plus de 4 atomes de carbone par molécule est très importante.

Les exemples suivants illustrent l'invention sans toutefois en limiter la portée.

### EXEMPLE 1 : CATALYSEURS COMPORTANT DE L'ACIDE SULFURIQUE

### PRÉPARATION DU CATALYSEUR A CONFORME À L'INVENTION :

On active 15 g de silice de volume poreux total égal à 2,2 cm³/g, de surface spécifique égale à 420 m²/g et de diamètre moyen des particules égal à 60 µm (1 µm = 10⁻⁶ m) par séchage à 150°C pendant 12 heures. La silice ainsi activée est conservée sous azote. On procède alors à une imprégnation à sec de 10 g de ladite silice par 40,5 g d'une solution d'acide sulfurique à 99,8 % en poids. Le solide ainsi obtenu appelé catalyseur A, possède une teneur pondérale en acide sulfurique égale à 80,2 % poids. Il est conservé à l'abri de l'humidité.

### PRÉPARATION DU CATALYSEUR B SELON LA DEMANDE DE BREVET EP-A-0539277 (COMPARATIF) :

On active 15 g de silice de volume poreux total égal à 0,78 cm³/g, de surface spécifique égale à 30 m²/g et de diamètre moyen des particules égal à 65 µm, par séchage à 150°C pendant 12 heures. La silice ainsi activée est conservée sous azote. On procède alors à une imprégnation à sec de 10 g de ladite silice par 14,35 g d'une solution d'acide sulfurique à 99,8 % en poids. Le solide ainsi obtenu appelé catalyseur B, possède une teneur pondérale en acide sulfurique égale à 58,9 % poids. Il est conservé à l'abri de l'humidité.

### PREPARATION DU CATALYSEUR C COMPARATIF :

On active 30 g de silice de volume poreux total égal à 0,32 cm³/g, de surface spécifique égale à 250 m²/g, et de diamètre moyen des particules égal à 59 µm, par séchage à 150°C pendant 12 heures. La silice ainsi activée est conservée sous azote. On réalise alors une imprégnation à sec de 26 g de ladite silice calcinée par 14 g d'une solution d'acide sulfurique de titre égal à 99,7% en poids. Le solide obtenu, appelé catalyseur C, possède une teneur pondérale en acide sulfurique égale à 35%. Il est conservé à l'abri de l'humidité.

### ALKYLATION DE L'ISOBUTANE PAR LE BUTENE-1 :

On introduit 20 g du catalyseur A, B ou C préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm³ d'isobutane sont alors ajoutés dans le réacteur contenant le catalyseur sous agitation (hélice), ledit réacteur étant immergé dans un bain froid à -5°C. On laisse sous agitation le système catalyseur + isobutane pendant une durée de 30 minutes afin d'homogénéiser la température.

On ajoute régulièrement 2,6 g de butène-1 par heure pendant une durée totale de 6 heures, la température du réacteur est maintenu à -5 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 1 ci-après. La conversion de l'oléfine est de 100 %.

**TABLEAU 1**

| **COMPOSÉS** | **CATALYSEUR A conforme à l'invention** | **CATALYSEUR B comparatif** | **CATALYSEUR C comparatif** |
|---|---|---|---|
| C₅-C₇ | 3,4 | 10,1 | 13 |
| TMPs | 86,6 | 71,16 | 54,4 |
| DMHs | 6,3 | 8,44 | 8,1 |
| C₉⁺ | 3,7 | 10,3 | 24,5 |
| TMPs : triméthylpentanes DMHs : diméthylhexanes | | | |

Ainsi, le catalyseur A selon la présente invention donne de meilleurs résultats que le catalyseur B comparatif, mais surtout de meilleurs résultats que le catalyseur C comparatif. c'est-à-dire que d'une pan l'alkylat obtenu par ledit catalyseur A contient plus de triméthylpentanes que l'alkylat obtenu par les catalyseurs B et C, et d'autre pan la quantité de produits lourds obtenus par l'utilisation du catalyseur A est plus faible que dans le cas de l'utilisation des catalyseurs B ou C.

### EXEMPLE 2 : CATALYSEURS COMPORTANT DE L'ACIDE SULFURIQUE ET DE L'ACIDE TRIFLUOROMETHANE SULFONIQUE

### CATALYSEUR COMPARATIF : CATALYSEUR D

On active 16 g de silice macroporeuse de surface spécifique égale à 40 m²/g, de volume poreux total égal à 1,2 cm³/g, et constituée principalement de grains sensiblement sphériques de diamètre moyen égal à 110 µm, par calcination sous air pendant 4 heures à 500 °C. La silice ainsi activée est conservée sous argon. On procède alors à une imprégnation à sec, et à l'abri de l'humidité, de 14 g de ladite silice déshydratée par 22 g du mélange constitué par :
- 18,7 g d'une solution contenant 99 % poids d'H₂SO₄ et 1 % poids d'eau,
- 3,3 g d'une solution contenant 97,8 % d'acide CF₃SO₃H et 2,2 % poids d'eau.

La composition pondérale de la phase acide est la suivante :

| | |
|---|---|
| - H₂SO₄ | 84,15 % |
| - CF₃SO₃H | 14,68 % |
| - H₂O | 1,17 % |

Le solide ainsi obtenu, catalyseur D, comprend donc 61 % poids de phase acide. Il est conservé sous argon à -18 °C.

### CATALYSEUR CONFORME A L'INVENTION : CATALYSEUR E

On active 16 g de silice macroporeuse de surface spécifique égale à 435 m²/g, de volume poreux total égal à 2,5 cm³/g, et constituée principalement de grains sensiblement sphériques de diamètre moyen égal à 70 µm, par calcination sous air pendant 4 heures à 500° C. La silice ainsi activée est conservée sous argon. On procède alors à une imprégnation à sec, et à l'abri de l'humidité, de 14 g de ladite silice déshydratée par 63,4 g du mélange constitué par :
- 53,89 g d'une solution contenant 99 % poids d'H₂SO₄ et 1 % poids d'eau,
- 9,51 g d'une solution contenant 97,8 % d'acide CF₃SO₃H et 2,2 % poids d'eau,

La composition pondérale de la phase acide est la suivante :

| | |
|---|---|
| - H₂SO₄ | 84,15 % |
| - CF₃SO₃H | 14,68 % |
| - H₂O | 1,17 % |

Le solide ainsi obtenu catalyseur E, comprend donc 81,9 % poids de phase acide. Il est conservé sous argon à - 18° C.

### RÉSULTATS DES TESTS D'ALKYLATION DE L'ISOBUTANE PAR LE BUTENE-1.

Les catalyseurs D et E sont utilisés pour alkyler l'isobutane par le butène-1 de façon à produire des paraffines ramifiées à hauts indices d'octane. Les catalyseurs D et E sont testés selon le même protocole opératoire qui est décrit ci-après.

On introduit 36 g du catalyseur D ou E dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de - 20 °C.

100 cm³ d'isobutane sont alors ajoutés dans le réacteur contenant le catalyseur sous agitation, ledit réacteur étant immergé dans un bain froid à - 6 °C. On laisse sous agitation le système catalyseur + isobutane pendant une durée de 30 minutes afin d'homogénéiser la température.

On ajoute en continu un mélange d'isobutane et de butène-1, contenant 20 % poids de butène-1, pendant une durée totale de 8 heures, la température du réacteur est maintenu à -5 °C pendant toute la durée de l'injection. Le débit volumique du butène-1 est égal à 10 ml/h.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 2 ci-après. La conversion de l'oléfine est de 100 %.

**TABLEAU 2**

| | **CATALYSEUR D (Comparatif)** | **CATALYSEUR E (Selon l'invention)** |
|---|---|---|
| C₅-C₇ | 2,5 | 1,8 |
| C₈ total | 93 | 95,1 |
| C₉⁺ | 4,5 | 3,1 |
| TMPs/C₈ | 94 | 94,1 |
| TMPs/C₈ : proportion de triméthylpentanes (isomères 224, 223, 234 et 233) dans la coupe C₈. | | |

### EXEMPLE 3 : CATALYSEURS COMPORTANT DE L'ACIDE SULFURIQUE ET DE L'ANHYDRIDE SULFURIQUE

### PRÉPARATION DU CATALYSEUR F (Comparatif)

On active 14 g de silice macroporeuse, de surface spécifique égale à 27 m²/g de volume poreux égal à 1 cm³/g et constituée principalement de grains sensiblement sphériques de diamètre moyen égal à 42 µm, par calcination sous air pendant 4 heures à 500°C. Le solide ainsi activé est conservé sous argon. On procède alors à une imprégnation à sec de 10 g du solide calciné par 7 cm³ du mélange constitué de 80% poids d'acide sulfurique (de titre 100 %) et 20% poids d'anhydride sulfurique. Le catalyseur ainsi obtenu, catalyseur F, contient 13,5 g d'oléum et 10 g de silice soit 57,4 % poids de phase acide, et est conservé sous atmosphère d'argon à -18 °C.

### PRÉPARATION DU CATALYSEUR G (Selon l'invention)

On active 14 g de silice macroporeuse, de surface spécifique égale à 456 m²/g de volume poreux égal à 2,1 cm³/g et constituée principalement de grains sensiblement sphériques de diamètre moyen égal à 42 µm, par calcination sous air pendant 4 heures à 500°C. Le solide ainsi active' est conservé sous argon. On procède alors à une imprégnation à sec de 10 g du solide calciné par 20 cm³ du mélange constitué de 80 % poids d'acide sulfurique (de titre 100 %) et 20% poids d'anhydride sulfurique. Le catalyseur ainsi obtenu, catalyseur G, contient 38,57 g d'oléum et 10 g de silice, soit 79,4 % poids de phase acide, et est conservé sous atmosphère d'argon à - 18 °C.

### ALKYLATION DE L'ISOBUTANE PAR LE BUTENE-1

On introduit 20 g du catalyseur F ou du catalyseur G dans un réacteur en verre du type Fischer & Porter d'un volume de 360 cm³ préalablement purgé sous débit d'argon. Le réacteur contenant le catalyseur est alors fermé puis mis sous vide primaire, puis il est refroidi à la température de -20°C.

72 cm³ d'isobutane sont alors ajoutés dans le réacteur contenant le catalyseur sous agitation, ledit réacteur étant immergé dans un bain froid à -20 °C. On laisse sous agitation le système catalyseur + isobutane pendant une durée de 30 minutes afin d'homogénéiser la température.

On ajoute régulièrement 135 cm³ d'un mélange constitué de 24 % en volume de butène-1 et de 76 % en volume d'isobutane, pendant une durée totale de 10 heures, la température du réacteur étant maintenue à -15 °C pendant toute la durée de l'injection.

Après réaction, la phase hydrocarbure est soutirée du réacteur, l'isobutane est évaporé lentement. On recueille l'alkylat qui est analysé par chromatographie en phase vapeur ; sa composition pondérale est donnée dans le tableau 3 ci-dessous :

**TABLEAU 3**

| | **CATALYSEUR F** (comparatif) | **CATALYSEUR G** (selon l'invention) |
|---|---|---|
| iC₅ | 1,1 | 1,1 |
| C₆ | 1,3 | 0,8 |
| C₇ | 2,1 | 1,4 |
| C₈ | 88,1 | 92,8 |
| C₉ | 1,6 | 0,8 |
| C₉⁺ | 5,3 | 3,1 |

Pour le catalyseur F, la conversion de l'oléfine, est de 98 %, le rendement de l'alkylation est de 200% par rapport à l'oléfine transformée, et la fraction C₈ contient 88,6 % poids de triméthylpentanes (isomères 224, 223, 234 et 233 dans la coupe C₈).

Pour le catalyseur G, la conversion de l'oléfine pour le catalyseur G est de 100 %, le rendement de l'alkylation est de 200 % par rapport à l'oléfine transformée et la fraction C₈ contient 91,8 % de triméthylpentanes (isomères 224, 223, 234 et 233 dans la coupe C₈).

## Revendications

1. Catalyseur comprenant de la silice et une phase acide comportant de l'acide sulfurique, la silice ayant été imprégnée par ladite phase acide et ledit catalyseur étant caractérisé en ce qu'il est constitué essentiellement de particules de diamètre moyen compris entre 0,1 et 150 µm, et en ce que la silice, avant son imprégnation par ladite phase acide, possède un volume poreux total supérieur à 1,5 cm³/g.

2. Catalyseur selon la revendication 1 tel que sa teneur pondérale en phase acide est supérieure à 70 %.

3. Catalyseur selon l'une des revendications 1 ou 2 tel que la silice possède, avant son imprégnation par ladite phase acide, un volume poreux total compris entre 1,5 et 6 cm³/g.

4. Catalyseur selon l'une des revendications 1 à 3 tel que ladite phase acide comprend au moins un additif choisi dans le groupe formé par H₃PO₄, B(OH)₃, HB(HSO₄)₄, BF₄H, FSO₃H, CF₃CO₂H, SbF₅, CF₃SO₃H et SO₃.

5. Catalyseur selon la revendication 4 tel que ledit additif est l'acide trifluorométhane sulfonique CF₃SO₃H.

6. Catalyseur selon la revendication 4 tel que l'additif est l'anhydride sulfurique SO₃.

7. Procédé de préparation d'un catalyseur selon l'une des revendications 1 à 6 tel que la silice est calcinée à une température supérieure à 50° C puis imprégnée par une phase acide comportant de l'acide sulfurique de concentration comprise entre 90 et 100% en poids.

8. Utilisation du catalyseur selon l'une des revendications 1 à 6 ou préparé selon la revendication 7 dans un procédé d'alkylation catalytique d'au moins un élément choisi dans le groupe formé par l'isobutane et l'isopentane par au moins un élément choisi dans le groupe formé par les oléfines comprenant de 3 à 6 atomes de carbone par molécule, dans laquelle la température de la réaction est inférieure à 10°C.

9. Utilisation selon la revendication 8 dans laquelle le catalyseur est mis en oeuvre dans une zone réactionnelle à mélange parfait ou s'en approchant.

10. Utilisation selon l'une des revendications 8 ou 9 dans laquelle le catalyseur est mis en oeuvre en lit mobile à co-courant.

## Claims

1. Catalyst containing silica and an acid phase consisting of sulphuric acid, the silica having been impregnated with said acid phase and said catalyst being characterised in that it is essentially composed of particles with an average diameter of between 0.1 and 150 µm, and in that the silica, prior to its impregnation with said acid phase, has a total porous volume greater than 1.5 cm³ per gram.

2. Catalyst according to claim 1 such that its content by weight of acid phase is greater than 70%.

3. Catalyst according to one of claims 1 or 2 such that the silica has, prior to its impregnation with said acid phase, a total porous volume of between 1.5 and 6 cm³ per gram.

4. Catalyst according to one of claims 1 to 3 such that said acid phase contains at least one additive selected from the group formed by H₃PO₄, B(OH)₃, HB(HSO₄)₄, BF₄H, FSO₃H, CF₃CO₂H, SbF₅, CF₃SO₃H and SO₃.

5. Catalyst according to claim 4 such that said additive is trifluoromethane sulphonic acid CF₃SO₃H.

6. Catalyst according to claim 4 such that the additive is sulphuric anhydride SO₃.

7. Process for preparation of a catalyst according to one of claims 1 to 6 such that the silica is calcined at a temperature greater than 50°C then impregnated with an acid phase containing sulphuric acid in a concentration of between 90 and 100% by weight.

8. Use of the catalyst according to one of claims 1 to 6 or prepared according to claim 7 in a process of catalytic alkylation of at least one element selected from the group formed by isobutane and isopentane by at least one element selected from the group formed by olefins containing 3 to 6 carbon atoms per molecule, in which the reaction temperature is lower than 10°C.

9. Use according to claim 8 in which the catalyst is used in a perfect or near-perfect mixture reaction zone.

10. Use according to one of claims 8 or 9 in which the catalyst is used in a co-flow fluidised bed.

## Patentansprüche

1. Katalysator, umfassend Kieselerde und eine saure Phase, welche Schwefelsäure umfaßt, wobei die Kieselerde durch die saure Phase imprägniert ist und der Katalysator dadurch gekennzeichnet ist, daß er im wesentlichen aus Teilchen mit einem mittleren Durchmesser von zwischen 0,1 und 150 µm gebildet ist und dadurch, daß die Kieselerde vor der Imprägnierung mit der sauren Phase ein Gesamtporenvolumen von über 1,5 cm³/g besitzt.

2. Katalysator nach Anspruch 1, dessen Gewichtsgehalt an saurer Phase über 70 % beträgt.

3. Katalysator nach einem der Ansprüche 1 oder 2, wobei die Kieselerde vor der Imprägnierung mit der sauren Phase ein Gesamtporenvolumen von einschließlich 1,5 und 6 cm³/g besitzt.

4. Katalysator nach einem der Ansprüche 1 bis 3, wobei die saure Phase wenigstens ein Additiv umfaßt, daß aus der Gruppe ausgewählt ist, die durch H₃PO₄, B(OH)₃, HB(HSO₄)₄, BF₄H, FSO₃H, CF₃CO₂H, SbF₅, CF₃SO₃H und SO₃ gebildet ist.

5. Katalysator nach Anspruch 4, wobei das Additiv Trifluormethansulfonsäure CF₃SO₃H ist.

6. Katalysator nach Anspruch 4, wobei das Additiv Schwefelsäureanhydrid SO₃ ist.

7. Verfahren zur Herstellung eines Katalysators gemäß einem der Ansprüche 1 bis 6, bei dem die Kieselerde bei einer Temperatur oberhalb von 50°C kalziniert, dann durch eine saure Phase imprägniert wird, welche Schwefelsäure mit einer Konzentration von zwischen einschließlich 90 und 100 Gew.% umfaßt.

8. Verwendung des Katalysators gemäß einem der Ansprüche 1 bis 6 oder hergestellt nach Anspruch 7 in einem Verfahren zur katalytischen Alkylierung von wenigstens einer Verbindung, die aus der Gruppe ausgewählt ist, die durch Isobutan und Isopentan gebildet ist, mit wenigstens einer Verbindung, welche aus der Gruppe ausgewählt ist, die durch die Olefine mit 3 bis 6 Kohlenstoffatome pro Molekül gebildet ist, wobei die Temperatur der Umsetzung unter 10°C liegt.

9. Verwendung nach Anspruch 8, wobei der Katalysator in einer Reaktionszone unter vollständiger oder angenähert vollständiger Durchmischung, eingesetzt wird.

10. Verwendung nach einem der Ansprüche 8 oder 9, wobei der Katalysator im Wirbelbett im Gleichstrom eingesetzt wird.
